(19) Europäisches Patentamt
European Patent Office
Office européen des brevets

(11) Publication number: **0 019 634**
**A1**

# (12) EUROPEAN PATENT APPLICATION

published in accordance with Art. 158(3) EPC

(21) Application number: **79900559.0**

(22) Date of filing: **14.05.79**

Data of the international application taken as a basis:

(86) International application number: **PCT/JP 79/00122**

(87) International publication number: **WO 79/01080 (13.12.79 79/25)**

(51) Int. Cl.³: **C 07 D 513/04,** A 61 K 31/495

(30) Priority: **17.05.78 JP 58656/78**
**29.08.78 JP 104437/78**
**20.10.78 JP 128557/78**

(43) Date of publication of application: **10.12.80**
**Bulletin 80/25**

(84) Designated Contracting States: **AT CH DE FR GB SE**

(71) Applicant: **TEIJIN LIMITED, 11, 1-Chome, Minamihonmachi Higashi-ku, Osaka-shi Osaka (JP)**

(72) Inventor: **KAYAMA, Yasutaka, 15-6, Tamadaira 5-chome, Hino-shi, Tokyo 191 (JP)**
Inventor: **HARA, Takeshi, 1214-1, Kunugida-cho, Hachioji-shi, Tokyo 193 (JP)**
Inventor: **SUZUKI, Yoji, 18-4, Tamadaira 3-chome, Hino-shi, Tokyo 191 (JP)**
Inventor: **SAITO, Masahiko, 876-2, Kitaakitsu, Tokorozawa-shi, Saitama 359 (JP)**

(74) Representative: **Grundy, Derek George Ritchie et al, CARPMAELS & RANSFORD 43, Bloomsbury Square, London WC1A 2RA (GB)**

## (54) HEXAHYDRONAPHTOIMIDAZOTHIAZOLES AND PROCESS FOR PREPARING SAME.

(57) Hexahydronaphtoimidazothiazoles represented by following general formula [I];

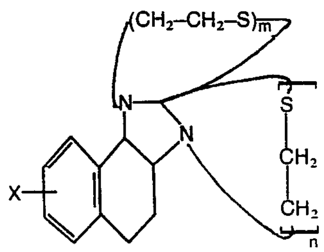

(wherein X represents hydrogen or halogen and m and n each differently represents 0 or 1), and the acid addition salts thereof. A process for preparing the compounds represented by general formula [I] comprises reacting a tetrahydronaphthoimidazoline-2-thione represented by the following general formula [II];

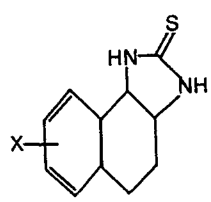

(wherein X represents hydrogen or halogen) with a compound represented by the following general formula [III];

$$Y{-}CH_2CH_2{-}Z$$

(wherein Y and Z are the same or different from each other, and each represents halogen, arenesulfonyloxy, or alkanesulfonyloxy group). These compounds show immunity-activating properties.

EP 0 019 634 A1

0019634

SPECIFICATION

TITLE MODIFIED
see front page

Title of the Invention

Hexahydronaphthoimidazothiazole and its derivatives and a method for preparing them.

Technical Field

The present invention relates to novel compounds useful as a medicine and veterinary drug and a method for preparing them. More particularly, the present invention relates to hexahydronaphthoimidazothiazole and its derivatives which are usable as a medicine having a function of immunostimulation or immunomodifying useful for the medical treatment of cancer or the remedy of auto-immunogenic diseases such as rheumatic arthritis and as a veterinary anthelmintic and a method for preparing them.

Background Art

Tetramisole, or 2,3,5,6-tetrahydro-6-phenylimidazo [2,1-b]thiazole hydro-chloride, is used as a veterinary anthelmintic and levamisole, or a left spiral isomer of tetramisole, is thoughtfully noted for use as a medicine for immunization therapy to control cancer or as a remedy for auto-immunogenic diseases such as rheumatic arthritis because of its

immunization activating function or immunization controlling function. And it is actually reported that levamisole is curative for such diseases as mentioned above to some degree; however, its remedial result is not always satisfactory.

The present inventors, in an effort to find out a compound having a better immunization activating function and immunization controlling function as compared with said levamisole, have synthesized many kinds of new compounds having a basic structure similar to that of levamisole, studied their pharmacological properties, and come to achieve the present invention.

Disclosure of Invention

The present invention relates to hexahydronaphthoimidazothiazole and its derivatives which may be expressed by the following formula [I]

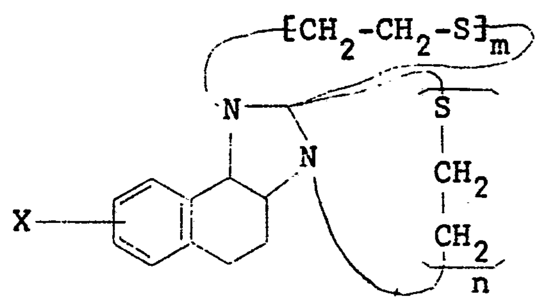

...... [I]

where X indicates hydrogen or halogen, m and n are different from each other and are 0 or 1.

According to the present invention, hexahydronaphthoimidazothiazole and its derivatives expressed

by the formula [I] can be prepared by reacting tetrahydronaphthoimidazoline-2-thion and its derivatives (3a,4,5,9b-tetrahydronaphtho[1,2-d]imidazoline-2-thion and its derivatives) which are expressed by the formula [II]

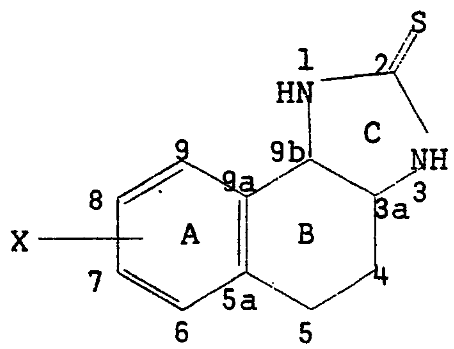
.......... [II]

where X indicates hydrogen or halogen, with a compound which is expressed by the formula [III]

$$Y\text{-}CH_2CH_2\text{-}Z \quad \ldots\ldots\ldots\ldots\ldots\ldots\ldots \quad [III]$$

where Y and Z are the same or different from each other and indicate halogen, allensulfonyloxy group or alkanesulfonyloxy group. A, B, and C are symbols to indicate the names of the respective rings and the configuration of the B ring and the C ring may be either trans form or cis form.

In the present invention, tetrahydronaphthoimidazoline-2-thion and its derivatives which are expressed by the formula [II] can be prepared by reacting a compound (1,2-diamino-1,2,3,4-tetrahydronaphthalene or its derivative) which is expressed by

the formula [IV]

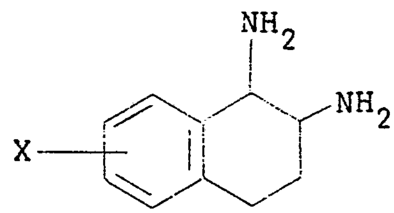

............. [IV]

where X indicates hydrogen or halogen, with carbon disulfide, followed by the reaction to remove hydrogen sulfide. In the compound according to the formula [IV], the configuration of the two amino groups may be either trans geometry or cis geometry.

Best Mode of Carrying Out the Invention

When m = 0 and n = 1 in the aforementioned formula [I], the compounds according to the present invention are 5,6,6a,8,9,11a-hexahydronaphtho-[1',2':4,5]imidazo[2,1-b]thiazole and its derivatives which are expressed by the following formula [I']

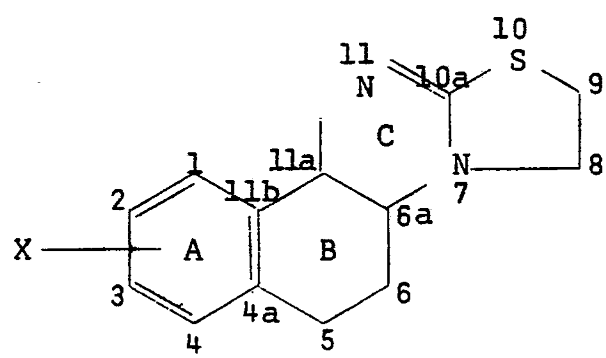

...... [I']

where X indicates hydrogen or halogen. A, B, and C are symbols to indicate the names of the respective rings.

When m = 1 and n = 0 in the formula [I], the compounds according to the present invention are 5,6,6a,9,10,11a-hexahydronaphtho[2',1':4,5]imidazo-[2,1-b]thiazole and its derivatives which are expressed by the following formula [I"]

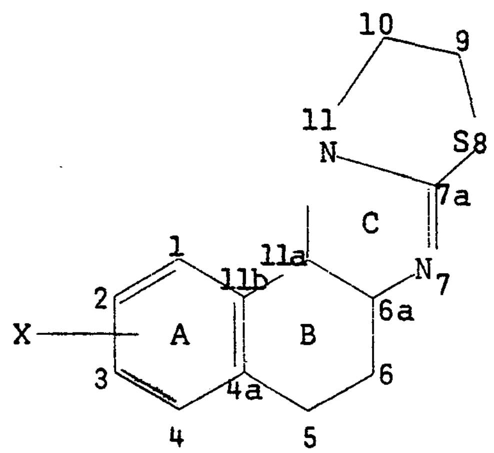

............. [I"]

where X indicates hydrogen or halogen.

The configuration of the B ring and the C ring of hexahydronaphthoimidazothiazole which is expressed by the abovementioned formula [I'] or [I"] may be either trans geometry or cis geometry. Also said hexahydronaphthoimidazothiazole and its derivatives may take a compound form having an acid addition salt of an inorganic acid or an organic acid. As for the inorganic acids, there are, for instance, hydrochloric acid, hydrobromic acid, hydriodic acid, sulfuric acid, and phosphoric acid. As for the organic acids, there are, for instance, acetic acid, propionic acid, glycollic acid, lactic acid, pyruvic acid, oxalic acid, malonic acid, succinic acid, maleic acid, fumaric acid, malic acid, tartaric acid, citric acid, benzoic acid,

cinnamic acid, mandelic acid, methanesulfonic acid, ethanesulfonic acid, hydroxy ethanesulfonic acid, benzenesulfonic acid, p-toluenesulfonic acid and salicylic acid. As for halogen which is used as a substituent group, fluorine, chlorine, or bromine substituted at the 1-, 2-, 3-, or 4-position is preferable.

When trans-3a,4,5,9b-tetrahydronaphtho[1,2-d]-imidazoline-2-thion or its derivative of the compounds which are expressed by the aforementioned formula [II] in the present invention is made to react with the compound which is expressed by the aforementioned formula [III], a mixture of trans-5,6,6a,8,9,11a-hexahydronaphtho[1',2':4,5]imidazo[2,1-b]thiazole and its derivatives which are expressed by the aforementioned formula [I'] and trans-5,6,6a,9,10,11a-hexahydronaphtho[2',1':4,5]imidazo[2,1-b]thiazole and its derivatives which are expressed by the aforementioned formula [I"] is obtained. The respective compounds can be isolated in the pure state from this mixture by any of the means, for instance, including extraction, chromatography, recrystallization, etc. When cis-3a,4,5,9b-tetrahydronaphtho[1,2-d]imidazoline-2-thion or its derivative of the compounds which are expressed by the aforementioned formula [II] is made to react with the compound which is expressed by the aforementioned formula [III], a mixture of the cis compound of formula [I'] and the cis compound of formula [I"] are obtained and the respective compounds can be

isolated in the pure state from the mixture according to the same method as mentioned above.

In the aforementioned formula [III], Y and Z are the same or different from each other and indicate a halogen atom, allensulfonyloxy group or alkanesulfonyloxy group. As for such halogen atom, chlorine, bromine and iodine are preferable. As for preferable examples of an allensulfonyloxy group and alkanesulfonyloxy group, a benzenesulfonyloxy group, p-toluenesulfonyloxy group, α-naphthalenesulfonyloxy group, β-naphthalenesulfonyloxy group, methanesulfonyloxy group, ethanesulfonyloxy group, etc. can be mentioned. To mention some of the compounds which are expressed by the formula [III] and are readily obtainable, there are 1,2-dichloroethane, 1-bromo-2-chloroethane, 1,2-dibromoethane, 1,2-diiodoethane, 2-chloroethyl p-toluenesulfonate, 2-bromoethyl p-toluenesulfonate, ethylenedi-p-toluenesulfonate, 2-bromoethylmethanesulfonate, etc.

In the reaction between the compound which is expressed by the aforementioned formula [II] and the compound which is expressed by the aforementioned formula [III], 0.8 to 10 moles of the compound expressed by the formula [III] is preferably used against 1 mole of the compound expressed by the formula [II]. In conducting a reaction, when the compound expressed by the formula [III] is liquid

under the reaction temperature, the compound expressed by the formula [II] may be allowed to react with the compound expressed by the formula [III] without the use of a solvent; however, the reaction is generally conducted in the presence of an appropriate solvent. As for such solvents, any solvent may be used unless it interferes with the reaction. To speak of appropriate solvents, for instance, such alcohols as ethanol, n-propanol, isopropanol, n-butanol, etc., such aromatic hydrocarbons as benzene, toluene, etc., such ethers as tetrahydrofuran, dioxane, etc., acetonitrile, N,N-dimethylformamide, etc. may be mentioned as readily obtainable ones. These reaction solvents may be used as a mixed solvent consisting of two or more solvents. The reaction temperature is in general appropriately selected from the range of 50°C to the boiling point of the reaction system. The reaction time varies depending upon the material compounds, scale of the reaction, reaction solvents, reaction temperature, etc.; however, it is usually in the range of 30 minutes to 48 hours.

The reaction can also be conducted in the presence of a base such as sodium hydrogencarbonate, potassium hydrogencarbonate, sodium carbonate, potassium carbonate, sodium hydroxide, etc.

In the present invention, tetrahydronaphtho-imidazoline-2-thion and its derivatives can be prepared by reacting (reaction A) 1,2-diamino-1,2,3,4-tetrahydronaphthalene or its derivative with carbon disulfide, followed by a reaction (reaction B) to remove hydrogen sulfide.

In the reaction A mentioned above, it is preferable to use 1 to 1.3 moles of carbon disulfide against 1 mole of 1,2-diamino-1,2,3,4-tetrahydronaphthalene and its derivatives. This reaction is generally conducted in the presence of an appropriate solvent. As for such solvents, any solvent may be used unless it interferes with the reaction. To speak of appropriate solvents, for instance, such alcohols as methanol, ethanol, n-propanol, isopropanol, n-butanol, etc., such lower carboxylic acid ester as ethyl acetate, etc., such halogenated hydrocarbon as methylene chloride, chloroform, 1,2-dichloroethane, etc., such aromatic hydrocarbons as benzene, toluene, etc., such ethers as ether, tetrahydrofuran, dioxane, etc., may be mentioned. These reaction solvents may be used as a mixed solvent consisting of two or more solvents. In case when the solvent is mixed uniformly with water, the solvent can be used in the reaction system in which water is contained in an amount not to break the uniformity. The optimum reaction temperature varies depending upon the used reaction solvent; however, the appropriate reaction temperature may usually be selected from the range of -10°C to the boiling point of the system. The reaction time varies depending upon the material compounds,

scale of the reaction, reaction solvents, reaction temperature, etc.; however, it is usually in the range of 10 minutes to 48 hours.

The compounds obtained from the reaction A are adducts of 1,2-diamino-1,2,3,4-tetrahydronaphthalene or its derivative and carbon disulfide. In some cases, the solubility of these compounds is lower than that of the material compounds depending upon the used reaction solvent and, as the reaction goes on, they tend to leave the reaction system and deposit in the form of a solid. They can, therefore, be isolated by filtration in such a case. The isolation can also be effected by evaporating and solidifying the reaction mixture. The adduct of 1,2-diamino-1,2,3,4-tetrahydronaphthalene or its derivative and carbon disulfide has enough purity to be subjected to the next reaction to remove hydrogen sulfide (reaction B).

The reaction B is usually conducted with the use of an appropriate reaction solvent while heating. As for such solvents, any solvent may be used unless it interferes with the reaction. To offer some examples of appropriate solvents, such alcohols as methanol, ethanol, n-propanol, isopropanol, n-butanol, etc., a mixture of any of these alcohols and water, such haloganated hydrocarbon as chloroform, 1,2-dichloroethane, etc. may be mentioned. The optimum reaction temperature varies depending upon the

used reaction solvent; the appropriate reaction temperature may ordinarily be selected from the range of 50°C to the boiling point of the system. The reaction time varies depending upon the material compounds used in the reaction A, scale of the reaction, reaction solvent, and reaction temperature; however, it is usually in the range of about 10 minutes to 24 hours.

The separation and purification of tetrahydro-naphthoimidazoline-2-thion and its derivatives, which are expressed by said formula [II] and obtained in the reaction B, from the reaction mixture can be effected by means of an ordinary operation such as filtration, recrystallization, chromatography, etc.

As for the reaction procedures for the reaction A and the reaction B, the adducts formed in the reaction A may be subjected to the reaction to remove hydrogen sulfide after their isolation; the reaction B may be conducted in succession to the reaction A without effecting the isolation of the adduct; or the reaction conditions under which the reaction B is conducted may be adopted while the reaction A is still going on, in which way the reaction A and the reaction B are substantially effected just as a single reaction instead of being carried on separately.

1,2-Diamino-1,2,3,4-tetrahydronaphthalene and its derivatives which are expressed by said formula

[IV] and are used as the material compounds in the process according to the present invention, can be prepared by various methods. To give examples of such methods, they can be prepared from 2-halo-3,4-dihydro-1(2H)-naphthalenon and its derivatives which are readily obtained from 3,4-dihydro-1(2H)-naphthalenon and its derivatives after the method published by Koptyung et al. in the Zhurnal Obshchei Khimii, Vol. 32, p.1613 (1962) and they can also be prepared from 1,2-dihydronaphthalene and its derivatives, which are derived from 3,4-dihydro-1(2H)-naphthalenon and its derivatives, after the method published by Swift et al. in the Journal of Organic Chemistry, Vol. 32, p.511 (1967).

The present invention is illustrated for further details but not limited by the following Examples.

Example 1

831 mg (5.12 m mol) of cis-1,2-diamino-1,2,3,4-tetrahydronaphthalene was dissolved in 16 ml of ethanol, to which 8 ml of water was added. 350 µl (5.84 m mol) of carbon disulfide was added to the mixture over a 1-minute interval with stirring at room temperature in an atmosphere of nitrogen. The admixture was heated under reflux for a period of 1 hour and a half. The reaction mixture was cooled on an ice-cold water bath to separate out crystals. The crystals were collected by filtration, washed with ether and dried under vacuum to obtain 803 mg (3.93 m

mol, yield 77%) of cis-3a,4,5,9b-tetrahydronaphtho-[1,2-d]imidazoline-2-thion. Upon recrystallization of thus obtained compound from aceton and ethanol, colorless needle crystals having a melting point of 237 to 237.5°C were obtained. The spectral data were as follows:

1R(KBr)$cm^{-1}$:

3190, 2930, 2900, 1530, 1475, 1441, 1291, 1255, 1204, 1186, 775, 748, 733, 692.

NMR($CDCl_3$:DMSO-$d_6$ = 5:1), δ from TMS (ppm):

1.69 - 2.09 (2H, m, $CH_2$ at the 4-position),
2.58 - 3.04 (2H, m, $CH_2$ at the 5-position),
4.44 (1H, dt, J=10 Hz and 4 Hz, CH at the 3a-position),
5.05 (1H, d, J=10 Hz, CH at the 9b-position),
7.15 - 7.38 (4H, m, H on the benzene ring),
7.79 (1H, broad s, NH),
8.03 (1H, broad s, NH).

Example 2

461 mg (2.84 m mol) of trans-1,2-diamino-1,2,3,4-tetrahydronaphthalene was dissolved in 8 ml of ethanol. 4 ml of water was added to the solution. 190 μl (3.17 m mol) of carbon disulfide was added thereto at room temperature with stirring and the mixture was heated under reflux for 4 hours. The reaction mixture was cooled on an ice-cold water bath to separate out crystals. The crystals were filtrated, washed with ether and dried under vacuum to give 350 mg (1.71 m mol, yield 60%) of trans-3a,4,5,9b-tetrahydronaphtho[1,2-d]imidazoline-2-thion. The spectral data were as follows:

IR (KBr) $cm^{-1}$:

3170, 2950, 2880, 1508, 1458, 1429, 1337, 1330, 1235, 1218, 1197, 1177, 1157, 1126, 1110, 742, 713, 689.

NMR ($CDCl_3$:DMSO-$d_6$=3:2), δ from TMS (ppm):

1.63 - 2.64 (2H, m, $CH_2$ at the 4-position),

2.90 - 3.67 (3H, m, CH at the 3a-position and $CH_2$ at the 5-position),

4.42 (1H, d, J=13 Hz, CH at the 9b-position),

7.13 - 7.50 (4H, m, H on the benzene ring),

8.47 (1H, broad s, NH),

9.08 (1H, broad s, NH).

Example 3

(1) 810 mg (4.99 m mol) of cis-1,2-diamino-1,2,3,4-tetrahydronaphthalene was dissolved in 16 ml of benzene, to which 340 µl (5.64 m mol) of carbon disulfide was added with stirring. The mixture was further stirred at room temperature for 2 hours. Separated crystals were collected by filtration, washed with benzene, and dried under vacuum to give 1.10 g (4.63 m mol, yield 93%) of an adduct of cis-1,2-diamino-1,2,3,4-tetrahydronaphthalene and carbon disulfide. The spectral data were as follows:

IR (KBr) $cm^{-1}$:

2940, 1490, 1450, 1300, 962.

(2) 820 mg (3.44 m mol) of the adduct of cis-1,2-diamino-1,2,3,4-tetrahydronaphthalene and carbon disulfide were suspended in 10 ml of ethanol and 5 ml of water. The suspension was heated under reflux with stirring for four hours (reaction to remove hydrogen sulfide). The reaction mixture was cooled. The separated crystals were collected by filtration, washed with ethanol-ether, and dried under vacuum to give 464 mg (2.27 m mol, yield 66%) of cis-3a,4,5,9b-tetrahydronaphtho[1,2-d]imidazoline-2-thion. This compound showed the same spectral data as the compound obtained in Example 1.

Example 4

4.65 g (23.6 m mol) of cis-1,2-diamino-6-chloro-1,2,3,4-tetrahydronaphthalene was dissolved in 70 ml of ethanol. When 1.6 ml (26.7 m mol) of carbon disulfide was added to the solution at room temperature in an atmosphere of nitrogen with stirring, a white solid substance immediately started to precipitate. After the stirring was continued for another 10 minutes, 35 ml of water was added and the system was heated under reflux on an oil bath for 2 hours. After that the reaction mixture was cooled and 50 ml of ethyl ether was added and the precipitated crystals were collected by filtration. The collected crystals were washed with ether and dried under vacuum to give 3.99 g (yield 71%) of cis-7-chloro-3a,4,5,9b-tetrahydronaphtho[1,2-d]imidazoline-2-thion in the form of a white solid substance. This was recrystallized from acetone-acetonitrile to offer clorless prisms which have a melting point of 249 to 250°C. The spectral data were as follows:

IR (KBr) $cm^{-1}$:

3180, 2920, 1599, 1533, 1482, 1438, 1300, 1267, 1250, 1202, 825.

NMR (DMSO-$d_6$), δ from TMS (ppm):

1.54 - 1.84 (2H, m, $CH_2$ at the 4-position),
2.44 - 2.77 (2H, m, $CH_2$ at the 5-position),
4.34 (1H, dt, J=10 Hz, 5 Hz, CH at the 3a-position),
4.97 (1H, d, J=10 Hz, CH at the 9b-position),
7.24 - 7.46 (3H, m, H on the benzene ring),
8.36 (1H, broad s, NH),
8.70 (1H, broad s, NH).

Example 5

5.14 g (26.1 m mol) of trans-1,2-diamino-6-chloro-1,2,3,4-tetrahydronaphthalene was dissolved in 60 ml of ethanol, to which solution 1.70 ml (28.4 m mol) of carbon disulfide was added at room temperature in an atmosphere of nitrogen with stirring. About one minute after such addition, a solid substance started to precipitate. After stirring for another 10 minutes at room temperature, 30 ml of water was added, and the system was heated under reflux for 2.5 hours. Then the reaction mixture was cooled on an ice bath for 1 hour and the precipitated solid substance was

collected by filtration. This solid substance was washed with approximately 150 ml of water, was then washed with 20 ml of a mixture of ether and n-hexane (volume ratio 1:2), was further washed with 40 ml of n-hexane. It was dried under vacuum overnight to give 4.86 g (20.4 m mol, yield 78%) of trans-7-chloro-3a,4,5,9b-tetrahydronaphtho[1,2-d]imidazoline-2-thion. This was recrystallized from methanol and acetonitrile to offer colorless prisms having a melting point of 269 to 271°C (decomposition). The spectral data were as follows:

IR (KBr) $cm^{-1}$:

3160, 2930, 2870, 1600, 1515, 1485, 1337, 1228, 1196, 1176, 1147, 1089, 881, 823, 792, 682.

NMR ($DMSO-d_6$), δ from TMS (ppm):

1.60 - 2.30 (2H, m, $CH_2$ at the 4-position),
2.87 - 3.17 (2H, m, $CH_2$ at the 5-position),
3.32 - 3.59 (1H, m, CH at the 3a-position),
4.38 (1H, d, J=13 Hz, CH at the 9b-position),
7.17 - 7.50 (3H, m, H on the benzene ring),
8.73 (1H, broad s, NH),
9.18 (1H, broad s, NH).

Example 6

3.84 g (21.3 m mol) of trans-1,2-diamino-6-fluoro-1,2,3,4-tetrahydronaphthalene was dissolved in 40 ml of ethanol, to which 1.4 ml (23.4 m mol) of carbon disulfide was added. Thus obtained white turbide solution was stirred for a while and 20 ml of

water was added thereto, which mixture was heated under reflux for 30 minutes. Thereafter the reaction mixture was cooled on an ice-cold water bath and the precipitated solid substance was collected by filtration. The obtained solid substance was first washed with 60 ml of water (three 20 ml portions), then with 20 ml of ethyl ether, and finally with 50 ml of n-hexane, and was dried under vacuum to give 3.88 g (17.4 m mol, yield 82%) of trans-7-fluoro-3a,4,5,9b-tetrahydronaphtho[1,2-d]imidazoline-2-thion in the form of a yellowish gray solid substance. This was recrystallized from aceton and acetonitrile to give light yellow needles having a melting point of 256.5 to 257.5°C (decomposition). The spectral data were as follows:

IR (KBr) $cm^{-1}$:

3430, 3165, 1620, 1501, 1437, 1422, 1337, 1252, 1230, 1208, 1193, 1158, 1100, 878, 857, 802, 731, 708, 689.

NMR (DMSO-$d_6$), δ from TMS (ppm):

1.63 - 2.27 (2H, m, $CH_2$ at the 4-position),
2.87 - 3.57 (3H, m, $CH_2$ at the 5-position and CH at the 3a-position),
4.35 (1H, d, J=12 Hz, CH at the 9b-position),
6.83 - 7.51 (3H, m, H on the benzene ring),
8.66 (1H, broad s, HN),
9.13 (1H, broad s, NH).

Example 7

4.30 g (17.8 m mol) of cis-1,2-diamino-7-bromo-1,2,3,4-tetrahydronaphthalene was dissolved in 86 ml of ethanol, to which 1.2 ml (20 m mol) of carbon disulfide was added at room temperature in an atmosphere of nitrogen with stirring. Stirring was continued for another 10 minutes. 43 ml of water was added to the mixture and was heated under reflux on an oil bath for 2.5 hours. The reactioh mixture was treated thereafter according to Example 4 to obtain 3.76 g (13.3 m mol, yield 75%) of cis-8-bromo-3a, 4, 5,9b-tetrahydronaphtho[1,2-d]imidazoline-2-thion in the form of light brown solid substance. This was recrystallized from methanol and acetonitrile to give colorless prisms having a melting point of 247 to 248°C. The spectral data were as follows:

IR (KBr) $cm^{-1}$:

3210, 2940, 1528, 1483, 1470, 1440, 1253, 1210, 1198, 1181, 831, 681.

NMR (DMSO-$d_6$), δ from TMS (ppm):

1.47 - 1.96 (2H, m, $CH_2$ at the 4-position),
2.35 - 2.77 (2H, m, $CH_2$ at the 5-position),
4.31 (1H, dt, J=10 Hz, 5 Hz, CH at the 3a-position),
4.94 (1H, d, J=10 Hz, CH at the 9b-position),
7.17 (1H, d, J=8 Hz, H at the 6-position),
7.43 (1H, dd, J=8 Hz, 2 Hz, H at the 7-position),
7.59 (1H, d, J=2 Hz, H at the 9-position),
8.33 (1H, broad s, NH),
8.64 (1H, broad s, NH).

Example 8

A mixture consisting of 723 mg (3.54 m mol) of trans-3a,4,5,9b-tetrahydronaphtho[1,2-d]imidazoline-2-thion, 1.77 g (6.34 m mol) of 2-bromoethyl p-toluenesulfonate, 795 mg (7.50 m mol) of sodium carbonate, and 13 ml of isopropanol was heated under reflux with stirring for 15 hours. The reaction mixture was cooled to room temperature and the obtained solid substance was separated by filtration and was washed with methanol. The solvent was removed from the mixture of the filtrate and the wash by distillation under decreased pressure. 20 ml of methylene chloride, 10 ml of water and 10 ml of 10% aqueous ammonia were added to the residue to effect its distribution among them. The water layer was further subjected to extraction with the use of methylene chloride. The layers of methylene chloride thus obtained were washed together with salt water and was dried with sodium sulfate anhydride. The residue which remained after the removal of the solvent by distillation was subjected to column chromatography on silica gel (100 g of silica gel) through which 100 ml of benzene, 100 ml of a mixture of benzene and ethyl acetate (volume ratio 4:1), 400 ml of a mixture of benzene and ethyl acetate (volume ratio 3:2), 600 ml of a mixture of benzene and ethyl acetate (volume ratio 2:3), and 1.2 ℓ of ethyl acetate were passed in this order. The flow was collected by 17-gram

fractions and the 25th to the 42nd fractions were mixed as a lot from which the solvent was removed by distillation under decreased pressure. The residue was dissolved in methylene chloride and was extracted from 4N hydrochloric acid. Then the layer of hydrochloric acid was made weakly basic with an aqueous solution of saturated sodium hydrogencarbonate and was extracted from methylene chloride. The extracted solution was washed with salt water and was dried with sodium sulfate anhydride. The residue which was left after the removal of the solvent by distillation was treated with a small amount of ether to give trans-5,6,6a,8,9,11a-hexahydronaphtho [1',2':4,5]imidazo[2,1-b]thiazole in the form of colorless crystals. This was recrystallized from methylenechloride-n-hexane to obtain colorless prisms having a melting point of 121.5 to 122.5°C. The spectral data were as follows:

IR (KBr) $cm^{-1}$:

1558, 1309, 1196, 1136, 1010, 741.

NMR ($CDCl_3$), δ from TMS (ppm):

1.69 - 3.99 (9H, m),

4.86 (1H, d, J=14 Hz),

7.01 - 7.22 (3H, m),

7.45 - 7.73 (1H, m).

Mass (70 eV), m/e:

230 ($M^+$).

The solvent was removed under decreased

pressure from a combined lot of the 61st fraction through the 105th fraction collected from the aforementioned column chromatography. The residue was put through the same processes as mentioned above to obtain trans-5,6,6a,9,10,11a-hexahydronaphtho-[2',1':4,5]imidazo[2,1-b]thiazole in the form of a colorless crystal. This was recrystallized from methylene chloride-n-hexane to give colorless prisms having a melting point of 127 to 128.5°C. The spectral data were as follows:

IR (KBr) $cm^{-1}$:

2825, 1565, 1322, 1289, 1279, 1246, 1237, 1167, 1143, 1123, 742.

NMR ($DCDl_3$), δ from TMS (ppm):

1.59 - 4.19 (10H, m),

7.17 (4H, s).

Mass (70 eV), m/e:

231 (4.5%), 230 (24.5%, $M^+$), 229 (25%), 202 (6%), 128 (100%).

Elemental analysis ($C_{13}H_{14}N_2S$):

Theoretical values: C67.79%, H6.13%, N12.16%.

Found values: C67.84%, H6.90%, N12.05%.

Example 9

A mixture consisting of 493 mg (2.41 m mol) of trans-3a,4,5,9b-tetrahydronaphtho[1,2-d]imidazoline-2-thion, 1.78 g (4.81 m mol) of ethylenedi-p-toluene sulfonate, 509 mg (4.80 m mol) of sodium carbonate and 20 ml of isopropanol was heated under reflux with

stirring for 12 hours. The residue which remained after the removal of the solvent by distillation was distributed between 50 ml of benzene and 30 ml of water. The layer of benzene solution was separated and washed with salt water and dried with sodium sulfate anhydride. The benzene solution was concentrated to yield a solid substance. This solid substance was separated out by filtration. The filtrate was further concentrated and the resulting residue was put to column chromatography on silica gel as in the case of Example 8 to obtain trans-5,6,6a,8,9,11a-hexahydronaphtho[1',2':4,5]imidazo[2,1-b]thiazole and trans-5,6,6a,9,10,11a-hexahydronaphtho-[2',1':4,5]imidazo[2,1-b]thiazole.

Example 10

A mixture consisting of 500 mg (2.45 m mol) of trans-3a,4,5,9b-tetrahydronaphtho[1,2-d]imidazoline-2-thion, 1,2-dibromoethane (8.98 m mol), 924 mg (9.10 m mol) of sodium carbonate and 8 ml of isopropanol was heated under reflux with stirring for 17 hours. The residue which remained after the removal of the solvent by distillation was distributed between 50 ml of benzene and 40 ml of 10% aqueous ammonia. The layer of benzene solution was separated, washed with salt water, and dried with sodium sulfate anhydride. The residue which was obtained by the removal of the solvent by distillation was put to column chromatography on silica gel as in the case of Example 8 to

obtain trans-5,6,6a,8,9,11a-hexahydronaphtho[1',2':4,5]-imidazo[2,1-b]thiazole and trans-5,6,6a,9,10,11a-hexahydronaphtho[2',1':4,5]imidazo[2,1-b]thiazole.

Example 11

A mixture consisting of 5.00 g (24.5 m mol) of cis-3a,4,5,9b-tetrahydronaphtho[1,2-d]imidazoline-2-thion obtained in Example 1, 13.00 g (46.6 m mol) of 2-bromoethyl p-toluene sulfonate, 5.20 g (49.1 m mol) of sodium carbonate, and 100 ml of isopropanol was heated under reflux with stirring for 14 hours. After the reflux, the residue which was obtained by removal of the solvent by distillation under decreased pressure was distributed between 60 of methylene chloride and 50 ml of water for separation. The water layer was further extracted with 60 ml of methylene chloride (two 30 ml portions). The two layers of methylene chloride was washed with 50 ml of water and 30 ml of salt water and dried with sodium sulfate anhydride. After drying, the residue which was obtained by removing the solvent by distillation was put to colum chromatograph on silica gel. As for the columns, column A and column B, each filled with 300 g of silica gel, were used. Each column was leached with a sequence of 0.3 ℓ of methylene chloride, 1 ℓ of a mixture of methylene chloride and methanol (volume ratio 49:1), 0.6 ℓ of a mixture of methylene chloride and methanol (volume ratio 97:3), 0.8 ℓ of a mixture of methylene chloride and methanol (volume

ratio 19:1), and 0.4 ℓ of a mixture of methylene chloride and methanol (volume ratio 2:3). The initial 900 ml of the effluent was omitted and the rest of the eluting solution was collected by 20-gram fractions. From the 15th to the 30th fractions collected from the column A and from the 19th to the 35th fractions collected from the column B gave 5,6,6a,8,9,11a-hexahydronaphtho[1',2':4,5]imidazo-[2,1-b]thiazole in the form of a colorless crystal. This was further recrystallized from a mixture of methylene chloride and n-hexane to obtain colorless prisms having a melting point of 96 to 97.5°C. The spectral data obtained with this compound were as follows:

IR (KBr) $cm^{-1}$:

2930, 2830, 1596, 1235, 1218, 1182, 1162, 1155, 742.

NMR ($CDCl_3$), δ from TMS (ppm):

1.72 - 2.00 (2H, m, $CH_2$ at the 6-position),

2.25 - 3.64 (6H, m, $CH_2$ at the 5-, 8-, and 9-positions),

3.85 (1H, dt, J=9 Hz and 5 Hz, CH at the 6a-position),

5.37 (1H, d, J=9 Hz, CH at the 11a-position),

6.98 - 7.54 (4H, m, H at the 1 to 4-positions).

Mass ($M^+$):

Theoretical value: 230.0878.

Found value: 230.0909 ± 0.0069.

A mixture consisting of the 60th to the 111th

fractions collected from the column A and the 70th to the 122nd fractions collected from the column B had its solvent removed by distillation under decreased pressure. The residue was processed as mentioned above to obtain cis-5,6,6a,9,10,11a-hexahydronaphtho-[2',1':4,5]imidazo[2,1-b]thiazole in the form of a colorless crystal. This compound was recrystallized from methylene chloride and n-hexane to obtain colorless needles having a melting point of 100 to 101°C. The spectral data were as follows:

IR (KBr) $cm^{-1}$:

2950, 2840, 1596, 1262, 1224, 1170, 1150, 1047, 763, 746, 660.

NMR ($CDCl_3$), δ from TMS (ppm):

1.70 - 2.07 (2H, m, $CH_2$ at the 6-position),

2.58 - 3.65 (6H, m, $CH_2$ at the 5-, 9-, and 10-positions),

4.42 (1H, d, J=9 Hz, CH at the 11a-position),

4.70 (1H, dt, J=9 Hz, 5 Hz, CH at the 6a-position),

6.99 - 7.30 (4H, m, H at the 1- to 4-positions).

Elemental analysis ($C_{13}H_{14}N_2S$):

Theoretical values: C67.79%, H6.13%, N12.16%.

Found values: C67.56%, H5.94%, N12.06%.

Example 12

190 mg of cis-5,6,6a,8,9,11a-hexahydronaphtho-[1',2':4,5]imidazo[2,1-b]thiazole obtained in Example 11 was dissolved in 40 ml of benzene, to which solution a hydrogen chloride gas was introduced at

room temperature. Then a nitrogen gas was bubbled into the solution to remove excess hydrogen chloride gas and the solvent was removed by distillation under decreased pressure. The residue was crystallized from methanol and ether. The crystals were collected by filtration, washed with methanol and ether, and dried to obtain cis-5,6,6a,8,9,11a-hexahydronaphto-[1',2':4,5]imidazo[2,1-b]thiazole hydrochloride. This was recrystallized from methanol and acetonitrile to give colorless plates having a melting point of 232 to 235°C. The spectral data of this compound were as follows:

IR (KBr) $cm^{-1}$:

2920, 2750, 2640, 1588, 1528, 1487, 1449, 1435, 1237, 743.

NMR (DMSO-$d_6$), δ from TMS (ppm):

1.57 - 2.31 (2H, m, $CH_2$ at the 6-position),
2.40 - 2.84 (2H, m, $CH_2$ at the 5-position),
3.63 - 4.12 (4H, m, $CH_2$ at the 8- and 9-positions),
4.44 - 4.80 (1H, m, H at the 6a-position),
5.74 (1H, d, J=10 Hz, CH at the 11a-position),
7.17 - 7.53 (4H, m, H at the 1- to 4-positions),
12.03 (1H, broad).

Trans-5,6,6a,8,9,11a-hexahydronaphtho[1',2':4,5]imidazo[2,1-b]thiazole obtained in Example 8 was processed as same as above to obtain its hydrochloride compound. When recrystallized from methanol and acetonitrile, it gave colorless prisms having a

melting point of 254 to 259°C (decomposition). Its spectral data were as follows:

IR (KBr) $cm^{-1}$:

2650, 2600, 1527, 1499, 1490, 1452, 1331, 1240, 1233, 1220, 1202, 825, 754.

NMR (methanol-$d_4$), δ from TMS (ppm):

1.84 - 4.31 (9H, m, methylene at the 5-, 6-, 8- and 9-positions and methine at the 6a-position),

5.29 (1H, d, J=14 Hz, methine at the 11a-position),

7.15 - 7.34 (4H, m, hydrogen at the 1- to 4-positions).

Example 13

210 mg of cis-5,6,6a,9,10,11a-hexahydronaphto-[2',1':4,5]imidazo[2,1-b]thiazole obtained in Example 11 was dissolved in 6 ml of benzene, to which 2 ml of isopropanol saturated with hydrogen chloride was added and stirred for 1 hour. The solvent was removed from the solution by distillation under decreased pressure. 6 ml of ether and 2 ml of methanol were added to thus obtained residue and the formed crystals were separated by filtration to give cis-5,6,6a,9,10,11a-hexahydronaphtho[2',1':4,5]imidazo-[2,1-b]thiazole hydrochloride. This was recrystallized from methanol and acetonitrile to obtain colorless prisms having a melting point of 268 to 273°C (decomposition). The spectral data were as follows:

IR (KBr) $cm^{-1}$:

2750, 1594, 1517, 1432, 1270, 1194, 774, 754.

NMR ($DMSO-d_6$), δ from TMS (ppm):

1.57 - 2.23 (2H, m, $CH_2$ at the 6-position),

2.40 - 2.85 and 3.02 - 4.03 (6H, m, $CH_2$ at the 5-, 9- and 10 positions),

5.11 (1H, dd, J=10 Hz, 3 Hz, CH at the 6a-position),

5.27 (1H, d, J=10 Hz, CH at the 11a-position),

7.23 - 7.45 (4H, m, H at the 1- to 4-positions).

Elemental analysis ($C_{13}H_{15}ClN_2S$):

Theoretical values: C58.53%, H5.67%, N10.50%.

Found values: C58.36%, H5.72%, N10.87%.

Trans-5,6,6a,9,10,11a-hexahydronaphtho-[2',1':4,5]imidazo[2,1-b]thiazole obtained in Example 8 was processed as same as the preceding case to obtain its hydrochloride. When this was recrystallized from methanol and acetonitrile, colorless prisms having a melting point of 232 to 235°C were obtained. Its spectral data were as follows:

IR (KBr) $cm^{-1}$:

2920, 2750, 2640, 1588, 1528, 1487, 1449, 1435, 1273, 743.

NMR ($DMSO-d_6$), δ from TMS (ppm):

1.57 - 2.31 (2H, m, methylene at the 6-position),

2.40 - 2.84 (2H, m, methylene at the 5-position),

3.63 - 4.12 (4H, m, methylene at the 8- and 9-positions),

4.44 - 4.80 (1H, m, methine at the 6a-position),

5.74 (1H, d, J=10 Hz, methine at the 11a-position),

7.17 - 7.53 (4H, m, H at the 1- to 4-positions),

12.03 (1H, broad s, proton of hydrochloric acid).

Example 14

A mixture consisting of 3.00 g (12.6 m mol) of cis-7-chloro-3a,4,5,9b-tetrahydronaphtho[1,2-d]-imidazoline-2-thion, 7.01 g (25.1 m mol) of 2-bromoethyl p-toluenesulfonate, 1.50 g (14.2 m mol) of sodium carbonate anhydride and 100 ml of isopropanol was heated under reflux with stirring for 17.5 hours. The reaction mixture was cooled to room temperature. The solid substance was separated out by filtration and washed with 40 ml of water and 50 ml of methylene chloride. The filtrate and the wash were mixed, from which mixture the solvent was removed by distillation under decreased pressure. 30 ml of methylene chloride, 50 ml of water, and 10 ml of aqueous solution of saturated sodium hydrogencarbonate were added to the residue to effect fractional precipitation. The water layer was further subjected to extraction with 60 ml of methylene chloride (one 40 ml portion and one 20 ml portion, making two times). The layers of methylene chloride thus obtained were washed together first with an aqueous solution of saturated sodium hydrogencarbonate, then with saturated salt water, and was then dried with sodium sulfate anhydride. 4.12 g of high brown oily substance, which was obtained by removing the solvent by distillation under decreased pressure, was put to column chromatography on silica gel, in

which the elution was effected first with 0.3 ℓ of methylene chloride, then with 2 ℓ of a mixture of methanol and methylene chloride (volume ratio 2:98). The initial 900 ml of the effluent was omitted and the rest was collected by 20-gram fractions with a fraction collector. The solvents were removed from a collection of the 12th through the 19th fractions by distillation under decreased pressure to give cis-3-chloro-5,6,6a,8,9,11a-hexahydro-naphtho[1',2':4,5]imidazo[2,1-b]thiazole in the form of a light yellow crystal. Also when the solvents were removed from another collection of the 30th through the 56th fractions by distillation under decreased pressure, cis-3-chloro-5,6,6a,9,10,11a-hexahydronaphtho[2',1':4,5]imidazo[2,1-b]-thiazole was obtained in the form of a light yellow oily substance.

The former was recrystallized from methylene chloride and hexane to give colorless needles having a melting point of 95.5 to 96.5°C. Its spectral data were as follows:

IR (KBr) $cm^{-1}$:

1590, 1471, 1213, 1208, 1180, 1157, 1148, 872, 828, 802, 761.

NMR ($CDCl_3$), $\delta$ from TMS (ppm):

1.75 - 2.03 (2H, m, methylene at the 6-position),

2.26 - 3.72 (6H, m, methylene at the 5-, 8-, and 9-positions),

3.90 (1H, dt, J=9 Hz, 5 Hz, methine at the 6a-position),

5.40 (1H, d, J=9 Hz, methine at the 11a-position),

7.13 - 7.57 (3H, m, hydrogen at the 1- to 4-positions).

The spectral data of the latter were as follows:

IR (neat) $cm^{-1}$:

2930, 2840, 1690, 1600, 1480, 1438, 1280, 1266, 1223, 1190, 1170, 1090, 881, 678.

NMR ($CDCl_3$), δ from TMS (ppm):

1.67 - 2.19 (2H, m, $CH_2$ at the 6-position),

2.57 - 2.87 (2H, m, $CH_2$ at the 5-position),

3.02 - 3.63 (4H, m, $CH_2$ at the 9- and 10-positions),

4.45 (1H, d, J=9 Hz, CH at the 11a-position),

4.78 (1H, dt, J=9 Hz, 5 Hz, CH at the 6a-position),

7.02 - 7.41 (3H, m, H at the 1- to 4-positions).

Example 15

268 mg of cis-3-chloro-5,6,6a,9,10,11a-hexahydronaphtho[2',1':4,5]imidazo[2,1-b]thiazol obtained in Example 14 was dissolved in 10 ml of dry benzene. A hydrogen chloride gas was introduced into the solution placed on an ice-cold water bath with stirring for 15 minutes. Further a nitrogen gas was introduced for 20 minutes. The residue remaining after the removal of the solvent by distillation under decreased pressure was crystallized with ether to obtain cis-3-chloro-5,6,6a,9,10,11a-hexahydronaphtho-[2',1':4,5]imidazo[2,1-b]thiazole hydrochloride. This

was recrystallized from metanol and acetonitrile to obtain colorless prisms having a melting point of 263 to 269°C (decomposition). The spectral data were as follows:

IR (KBr) $cm^{-1}$:

2960, 2920, 2840, 2770, 1582, 1517, 1483, 1272, 1192, 818.

NMR (DMSO-$d_6$), δ from TMS (ppm):

1.5 - 2.2 (2H, m, $CH_2$ at the 6-position),
2.5 - 2.8 (2H, m, $CH_2$ at the 5-position),
3.2 - 4.3 (4H, m, $CH_2$ at the 9- and 10-positions),
5.09 - 5.52 (2H, m, 6a, CH at the lla-position),
7.33 - 7.66 (3H, m, H at the 1- to 4-positions).

Cis-3-chloro-5,6,6a,8,9,lla-hexahydronaphtho-[l',2':4,5]imidazo[2,l-b]thiazole obtained in Example 14 was processed as same as in the preceding case to obtain its hydrochloride. This was recrystallized from methanol and acetonitrile to give colorless prisms having a melting point of 234 to 239°C (decomposition). The spectral data was as follows:

IR (KBr) $cm^{-1}$:

2920, 2730, 2670, 2630, 1590, 1524, 1439, 1278, 1204, 872, 811.

NMR (DMSO-$d_6$), δ from TMS (ppm):

1.56 - 2.34 (2H, m, methylene at the 6-position),
2.44 - 2.84 (2H, m, methylene at the 5-position),
4.65 (lH, dt, J=10 Hz, 4 Hz, methine at the 6a-position),

5.81 (1H, d, J=10 Hz, methine at the 11a-position),

7.29 - 7.64 (3H, m, hydrogen at the 1- to 4-positions),

- 12.1 ppm (1H, broad peak, HCl).

Example 16

A mixture consisting of 3.00 g (12.6 m mol) of trans-7-chloro-3a,4,5,9b-tetrahydronaphtho[1,2-d]-imidazoline-2-thion obtained in Example 5, 7.00 g (25.1 m mol) of 2-bromoethyl p-toluenesulfonate, 1.50 g (14.2 m mol) of sodium carbonate anhydride and 100 ml of isopropanol was heated under reflux with stirring for 29.5 hours. Thereafter, this was subjected to substantially the same process as mentioned in Example 14 to obtain trans-3-chloro-5,6,6a,8,9,11a-hexahydro-naphtho[1',2':4,5]imidazo[2,1-b]thiazole and trans-3-chloro-5,6,6a,9,10,11a-hexahydronaphtho[2',1':4,5]-imidazo[2,1-b]thiazole.

The former was recrystallized from methylene chloride and hexane to give colorless plates having a melting point of 171.5 to 173°C. Its spectral data were as follows:

IR (KBr) $cm^{-1}$:

2830, 1563, 1469, 1311, 1274, 1204, 1198, 1182, 1139, 887, 828, 771.

NMR ($CDCl_3$), $\delta$ from TMS (ppm):

1.76 - 2.38 (2H, m, methylene at the 6-position),

2.54 - 3.10 (2H, m, methylene at the 5- and 9-positions (or at the 8-positions),

3.22 - 4.01 (3H, m, methylene at the 8-position

(or 9-position), methine at the 6-position),

4.83 ppm (1H, d, J=13 Hz, methine at the 11a-position),

7.13 - 7.67 (3H, m, hydrogen at the 1- to 4-position).

The spectral data of the latter were as follows:

NMR ($CDCl_3$), δ from TMS (ppm):

1.73 - 4.44 (10H, m),

7.19 (3H, s).

Example 17

Addition of hydrogen chloride was effected after the same method as adopted in Example 12 to 906 mg (3.42 m mol) of trans-3-chloro-5,6,6a,9,10,11a-hexahydronaphtho[2',1':4,5]imidazo[2,1-b]thiazole obtained in Example 16 to obtain 1.02 g of trans-3-chloro-5,6,6a,9,10,11a-hexahydronaphtho[2',1':4,5]-imidazo[2,1-b]thiazol hydrochloride. This was recrystallized from methanol and acetonitrile to give colorless needles which start to decompose gradually at 269°C. The spectral data were as follows:

IR (KBr) $cm^{-1}$:

2950, 2890, 2850, 2750 - 2700, 2670, 1601, 1544, 1488, 1366, 1338, 1264, 1167, 1128, 883, 824, 658.

NMR (DMSO-$d_6$), δ from TMS (ppm):

1.87 - 2.40 (2H, m, $CH_2$ at the 6-position),

2.94 - 3.17 (2H, m, $CH_2$ at the 5-position),

3.52 - 4.64 (5H, m, $CH_2$ at the 9- and 10-positions and CH at the 6a-position),

4.77 (1H, d, J=14 Hz, CH at the 11a-position),

7.27 - 7.74 (3H, m, H at the 1- to 4-positions).

Trans-3-chloro-5,6,6a,8,9,11a-hexahydronaphtho-[1',2':4,5]imidazo[2,1-b]thiazole obtained in Example 16 was subjected to the same process as mentioned above to obtain its hydrochloride. This was recrystallized from methanol and acetonitrile to give colorless needles having a melting point of 275 to 280°C (decomposition). Its spectral data were as follows:

IR (KBr) $cm^{-1}$:

2700, 2620, 1572, 1548, 1487, 1379, 1339, 1193, 1162, 828, 803.

NMR (DMSO-$d_6$), $\delta$ from TMS (ppm):

1.74 - 2.43 (2H, m, methylene at the 6-position),

2.96 - 3.17 (2H, m, methylene at the 5-position),

3.31 - 4.24 (5H, m, methylene at the 8- and 9-positions and methine at the 6a-position),

5.34 (1H, d, J=14 Hz, methine at the 11a-position),

7.27 - 7.67 (3H, m, hydrogen at the 1- to 4-positions).

Example 18

A mixture consisting of 2.00 g (9.00 m mol) of trans-7-fluoro-3a,4,5,9b-tetrahydronaphtho[1,2-d]-imidazoline-2-thion obtained in Example 6, 5.02 g (18.0 m mol) of 2-bromoethyl p-toluenesulfonate, 1.05 g (9.90 m mol) of sodium carbonate anhydride and 70 ml of isopropanol was heated under reflux with stirring for 38 hours. Then this was subjected to substantially the same process as mentioned in Example

14 to obtain trans-3-fluoro-5,6,6a,8,9,11a-hexahydronaphtho[1',2':4,5]imidazo[2,1-b]thiazole and trans-3-fluoro-5,6,6a,9,10,11a-hexahydronaphtho[2',1':4,5]-imidazo[2,1-b]thiazole.

The spectral data of the former were as follows:

IR (KBr) $cm^{-1}$:

3420, 2940, 2835, 1613, 1565, 1489, 1474, 1438, 1419, 1342, 1310, 1274, 1240, 1201, 1172, 1136, 1120, 1094, 1013, 907, 892, 810, 772.

NMR ($CDCl_3$), δ from TMS (ppm):

1.90 - 2.37 (2H, m, methylene at the 6-position),
2.54 - 3.72 (7H, m, methylene at the 5-, 8-, and 9-positions and methine at the 6a-position),
4.80 (1H, d, J=12 Hz, methine at the 11a-position),
6.74 - 7.72 (3H, m, hydrogen at the 1- to 4-positions).

The spectral data of the latter were as follows:

IR (KBr) $cm^{-1}$:

2950, 2860, 1615, 1575, 1492, 1346, 1322, 1278, 1254, 1219, 1168, 1149, 1121, 1068, 921, 912, 867.

NMR ($CDCl_3$), δ from TMS (ppm):

1.77 - 2.59 (2H, m, $CH_2$ at the 6-position),
2.73 - 4.10 (8H, m, $CH_2$ at the 5-, 9-, and 10-positions and CH at the 6a- and 11a-positions),
6.73 - 7.18 (3H, m, H at the 1- to 4-positions).

Example 19

Addition of hydrogen chloride was effected after

the same method as adopted in Example 12 to 340 mg (1.43 m mol) of trans-3-fluoro-5,6,6a,9,10,11a-hexahydronaphtho[2',1':4,5[imidazo[2,1-b]thiazole obtained in Example 18 to obtain trans-3-fluoro-5,6,6a,9,10,11a-hexahydronaphtho[2',1':4,5]imidazo-[2,1-b]thiazole hydrochloride. This was recrystallized from methanol and acetonitrile to give 250 mg (0.874 m mol) of colorless needles. The spectral data were as follows:

IR (KBr) $cm^{-1}$:

2940, 2880, 2825, 2705, 2655, 1616, 1586, 1549, 1493, 1459, 1418, 1362, 1337, 1268, 1231, 1175, 1159, 1128, 880, 817, 788, 659.

NMR ($DMSO-d_6$), δ from TMS (ppm):

1.97 - 2.50 (2H, m, $CH_2$ at the 6-position),

3.02 - 4.73 (8H, m, $CH_2$ at the 5-, 9-, and 10-positions and CH at the 6a- and 11a-positions),

6.88 - 7.15 (2H, m, H at the 2- and 4-position),

7.35 - 7.60 (1H, m, H at the 1-position).

Trans-3-fluoro-5,6,6a,8,9,11a-hexahydronaphtho[1',2':4,5]imidazo[2,1-b]thiazole obtained in Example 18 was subjected to the same process as mentioned above to obtain its hydrochloride. The spectral data were as follows:

IR (KBr) $cm^{-1}$:

2865, 2755, 2575, 1617, 1529, 1495, 1461, 1421, 1376, 1331, 1229, 1213, 1164, 1133, 1102, 1073,

875, 840, 830.

NMR (DMSO-$d_6$), δ from TMS (ppm):

1.76 - 2.54 (2H, m, methylene at the 6-position),

2.97 - 4.96 (7H, m, methylene at 5-, 8-, and 9-positions and methine at the 6a-position),

5.30 (1H, d, J=13 Hz, methine at 11a-position),

6.95 - 7.83 (3H, m, hydrogen at 1- to 4-positions).

Example 20

This example shows the immunization activating function of cis-5,6,6a,8,9,11a-hexahydronaphtho[1',2':4,5]imidazo[2,1-b]thiazole hydrochloride (hereinafter called drug A) and trans-5,6,6a,8,9,11a-hexahydronaphtho[1',2':4,5]imidazo[2,1-b]thiazole hydrochloride (hereinafter called drug B) which were obtained in Example 12 and cis-5,6,6a,9,10,11a-hexahydronaphtho-[2',1':4,5]imidazo[2,1-b]thiazole hydrochloride (hereinafter called drug C) and trans-5,6,6a,9,10,11a-hexahydronaphtho[2',1':4,5]imidazo[2,1-b]thiazole hydrochloride (hereinafter called drug D) which were obtained in Example 13.

(1) Effect of humoral antibody formation

Experiment-1: ICR mice (male, 8 weeks old) or $CDF_1$ mice (male, 8 weeks old) were administered intraperitoneally with $10^8$ sheep red blood cells as antigen and 24 hours after the administration of antigen, the respective drugs were likewise administered intraperitoneally. 4 days after the administration of antigen, antibody-forming cells

in the spleens of the mice were assayed according to the method of A.J. Cunningham and A. Czenberg (see the Immunology, Vol.14, pp599 - 600, 1969). For purposes of comparison, experiments were also conducted with the mice which were not administered with the drugs and with those which were administered with publicly known levamisole in the place of the drugs according to the present invention. The results of these experiments are shown in Table 1 in which the results obtained with the ICR mice are given in Experiment-1a and those obtained with the $CDF_1$ mice are given in Experiment-1b.

Experiment-2: C3H/He mice (male, 17 weeks old) were administered intravenously with $5 \times 10^5$ sheep red blood cells as antigen and 24 hours after the administration of antigen, the respective drugs were administered hypodermically in the inquinal region of the mice. 4 days after the aministration of antigen, antibody-forming cells in the spleens of the mice were assayed according to the same method as adopted in Experiment-1. As was in the case of Experiment-1, the experiment without the use of the drugs and the experiment with the use of levamisole were also conducted for purposes of comparison. The results of these experiments are shown in Table 1.

Table 1

| Drug | Dosage (mg/mouse) | No. of antibody-forming cells/$10^6$ spleen cells | | |
|---|---|---|---|---|
| | | Experiment-1a | Experiment-1b | Experiment-2 |
| No drug | 0 | 1500 ± 170 | 930 ± 90 | 750 ± 140 |
| Levamisole | 0.1 | 3530 ± 260 | 1180 ± 120 | 1410 ± 420 |
| " | 0.4 | 1490 ± 140 | 1040 ± 160 | 860 ± 130 |
| Drug A | 0.1 | - | 1220 ± 100 | - |
| " | 0.4 | - | 860 ± 60 | - |
| Drug B | 0.1 | - | 1100 ± 100 | - |
| " | 0.4 | - | 1500 ± 200 | - |
| Drug C | 0.1 | 1800 ± 150 | - | 1000 ± 170 |
| " | 0.4 | 1610 ± 180 | - | 1265 ± 200 |
| Drug D | 0.1 | 2440 ± 160 | - | - |
| " | 0.4 | 2580 ± 220 | - | - |

As shown in Table 1, it is apparent that the drugs according to the present invention have an effect of remarkably increasing the number of the antibody-forming cells of the mice; i.e. they have an outstanding function to activate immunization.

(2) Effect on cellular immunity

The effect of the drugs on cellular immunity was estimated with an index of delayed-type hypersensitivity which is caused by injecting sheep red blood cells as antigen into the hind footpad of the mice (see the method described by P.H. Lagrange, G.B. Mackaness and T.E. Mille in the Journal of Experimental Medicine, Vol. 139, pp 1529 - 1539, 1974).

$10^8$ Sheep red blood cells suspended in 0.05 ml of saline buffered with phosphate were injected hypodermically into the hind footpad of ICR mice (male, 12 weeks old) and then the mice were administered intraperitoneally with the drugs. Four days after the administration of the drugs, $10^8$ sheep red blood cells were injected hypodermically into the hind footpad opposite to the one which was previously administered with antigen. 24 hours after the injection, the thickness of the hind footpad was measured with a dial thickness meter (G1 type, manufactured by Ozaki Seisakusho) to dermine the increase in thickness as compared with the uninjected hind footpad. For purposes of

comparison, experiments were conducted with the mice which were not given the drugs and with those which were administered with levamisole instead of the drugs of the present invention. The results of these experiments are shown in Table 2.

Table 2

| Drug | Dosage (mg/mouse) | Increase in thickness of footpad (mm) |
|---|---|---|
| No drug | 0 | 0.28 ± 0.04 |
| Levamisole | 0.1 | 0.33 ± 0.04 |
| " | 0.4 | 0.29 ± 0.04 |
| Drug A | 0.1 | 0.41 ± 0.04 |
| " | 0.4 | 0.44 ± 0.06 |
| Drug B | 0.1 | 0.57 ± 0.03 |
| " | 0.4 | 0.42 ± 0.04 |
| Drug C | 0.1 | 0.37 ± 0.05 |
| " | 0.4 | 0.38 ± 0.05 |
| Drug D | 0.1 | 0.37 ± 0.06 |
| " | 0.4 | 0.39 ± 0.04 |

It is clear from Table 2 that the drugs according to the present invention have an effect to increase the thickness of the footpad of the mice or, in other words, to enhance the cellular immunity equal to or more than levamisole.

## Industrial Application

Since hexahydronaphthoimidazothiazole and its derivatives have an excellent function of activating immunization or of controlling immunization, they have expected applications as a cancer remedy for immunization therapy or as a remedy for such auto-immunogenic diseases as rheumatic arthritis, etc. Also these compounds can be used as an anthelmintic for animals. Tetrahydronaphthoimidazoline-2-thion and its derivatives obtained according to the present invention are useful compounds as intermediary syntheses of hexahydronaphthoimidazothiazole.

0019634

CLAIMS

1. Hexahydronaphthoimidazothiazole and its derivatives which are expressed by the formula [I]

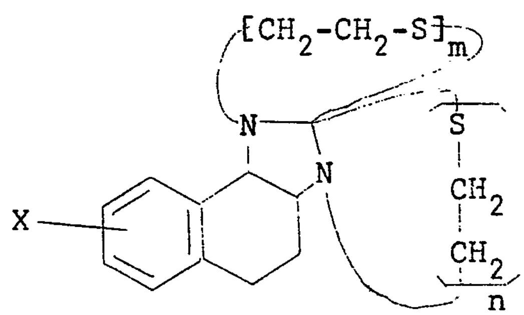

.........[I]

[where X indicates hydrogen or halogen: m and n differ from each other and are either 0 or 1 respectively].

2. Hexahydronaphthoimidazothiazole and its derivatives according to Claim 1 which are expressed by the formula [I']

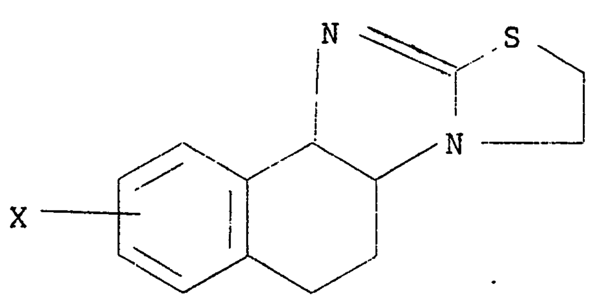

...... [I']

[where X indicates hydrogen or halogen].

3. Hexahydronaphthoimidazothiazole and its derivatives according to Claim 1 which are expressed by the formula [I"]

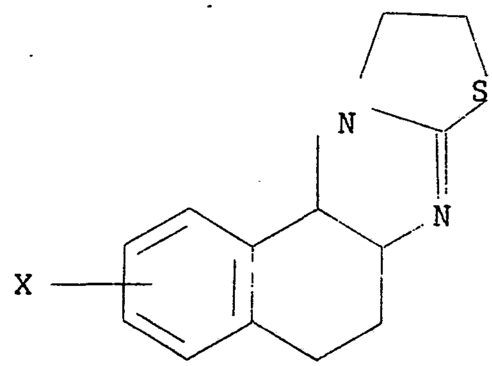

........ [I"]

[where X indicates hydrogen and halogen].

4. Hexahydronaphthoimidazothiazole according to Claim 1, wherein a compound expressed by the formula [I] forms an acid addition salt with an inorganic or organic acid.

5. A method for preparing hexahydronaphthoimidazothiazole expressed by the formula [I]

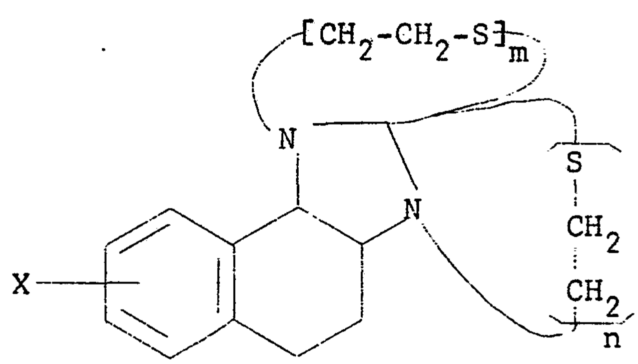

..... [I]

[where X indicates hydrogen or halogen: m and n differ

from each other and are either 0 or 1 respectively], which method comprises making tetrahydronaphtho-imidazoline-2-thion or its derivative expressed by the formula [II]

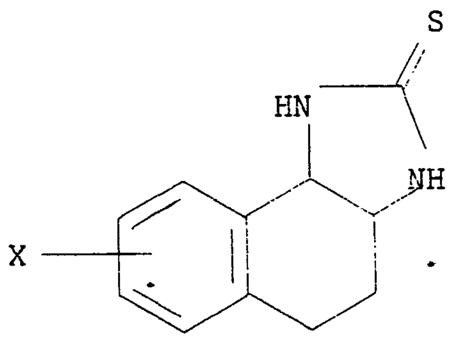 ........ [II]

[where X indicates hydrogen or halogen], react with a compound expressed by the formula [III]

$$Y\text{-}CH_2CH_2\text{-}Z \quad \ldots\ldots\ldots\ldots\ldots\ldots\ldots \quad [III]$$

[where Y and Z are the same or differ from each other and indicate halogen, allensulfonyloxy group, or alkanesulfonyloxy group respectively].

6. Tetrahydronaphthoimidazoline-2-thion and its derivatives expressed by the formula [II]

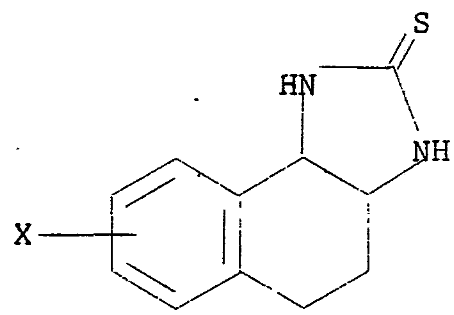

........ [II]

[where X indicates hydrogen or halogen].

7. A method for preparing tetrahydronaphtho-imidazoline-2-thion and its derivatives expressed by the formula [II]

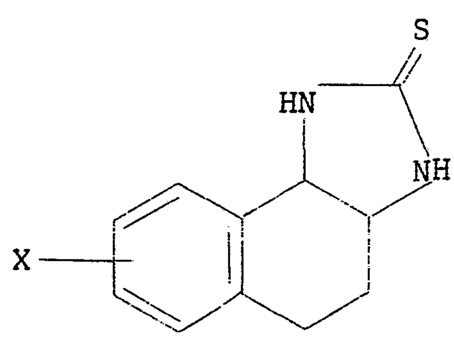

.......... [II]

[where X indicates hydrogen or halogen],
which method comprises making a compound expressed by the formula [IV]

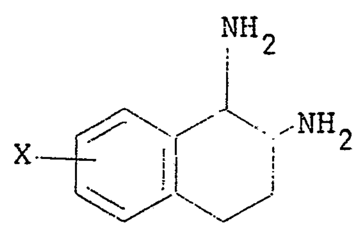

............... [IV]

[where X indicates hydrogen or halogen]

react with carbon disulfide, followed by the removal of hydrogen sulfide.

# INTERNATIONAL SEARCH REPORT

0019634

International Application No PCT/JP79/00122

**I. CLASSIFICATION OF SUBJECT MATTER** (if several classification symbols apply, indicate all) [3]

According to International Patent Classification (IPC) or to both National Classification and IPC

C07D 513/04,
A61K 31/495

**II. FIELDS SEARCHED**

Minimum Documentation Searched [4]

| Classification System | Classification Symbols |
|---|---|
| I P C | C07D 513/04, C07D 235/28 |
| I P C | A61K 31/495 |

Documentation Searched other than Minimum Documentation to the Extent that such Documents are Included in the Fields Searched [5]

**III. DOCUMENTS CONSIDERED TO BE RELEVANT** [14]

| Category * | Citation of Document, [16] with indication, where appropriate, of the relevant passages [17] | Relevant to Claim No. [18] |
|---|---|---|
| A | JP, A, 50-76090, 1975-6-21, See formula II | 1, 3, 4 |
| A | JP, A, 48-78170, 1973-10-20, See Page 1 | 6, 7 |

* Special categories of cited documents: [15]

"A" document defining the general state of the art

"E" earlier document but published on or after the international filing date

"L" document cited for special reason other than those referred to in the other categories

"O" document referring to an oral disclosure, use, exhibition or other means

"P" document published prior to the international filing date but on or after the priority date claimed

"T" later document published on or after the international filing date or priority date and not in conflict with the application, but cited to understand the principle or theory underlying the invention

"X" document of particular relevance

**IV. CERTIFICATION**

| Date of the Actual Completion of the International Search [2] | Date of Mailing of this International Search Report [2] |
|---|---|
| July 27, 1979 (27.07.79) | August 6, 1979 (06.08.79) |
| International Searching Authority [1] | Signature of Authorized Officer [20] |
| Japanese Patent Office | |